(19) Europäisches Patentamt
European Patent Office
Office européen des brevets

(11) EP 2 402 462 A1

(12) EUROPEAN PATENT APPLICATION

(43) Date of publication:
**04.01.2012 Bulletin 2012/01**

(51) Int Cl.:
***C12Q 1/68*** *(2006.01)*

(21) Application number: **11181688.0**

(22) Date of filing: **06.06.2008**

(84) Designated Contracting States:
**AT BE BG CH CY CZ DE DK EE ES FI FR GB GR HR HU IE IS IT LI LT LU LV MC MT NL NO PL PT RO SE SI SK TR**

(62) Document number(s) of the earlier application(s) in accordance with Art. 76 EPC:
**08104295.4 / 2 130 929**

(71) Applicants:
- **F. Hoffmann-La Roche AG**
  **4070 Basel (CH)**
  Designated Contracting States:
  **AT BE BG CH CY CZ DK EE ES FI FR GB GR HR HU IE IS IT LI LT LU LV MC MT NL NO PL PT RO SE SI SK TR**
- **Roche Diagnostics GmbH**
  **68305 Mannheim (DE)**
  Designated Contracting States:
  **DE**

(72) Inventors:
- **Sizmann, Dorothea**
  **82393 Iffeldorf (DE)**
- **Babiel, Reiner**
  **82418 Seehausen (DE)**
- **Glaubitz, Joachim**
  **5643 Sins (CH)**
- **Guertler, Lutz**
  **82166 Gräfelfing (DE)**
- **Young, Karen Kwok Ying**
  **San Ramon, CA 94583 (US)**

(74) Representative: **Herren, Barbara**
**Roche Diagnostics AG**
**Patent Department**
**Forrenstrasse**
**6343 Rotkreuz (CH)**

Remarks:
This application was filed on 16-09-2011 as a divisional application to the application mentioned under INID code 62.

# (54) Internally controlled multiplex detection and quantification of microbial nucleic acids

(57) The present invention relates to new methods and uses for the detection and quantification of nucleic acids of HIV, employing an internal quantitative reference. Preferred methods are based on the amplification of nucleic acids, preferably the polymerase chain reaction. Further provided are kits comprising components for performing said methods and uses.

Figure 4

| | Test 2 (GAG) | Test 2a (LTR) | Test 2 modified (LTR+GAG) |
|---|---|---|---|
| LOD [cp/ml] (by PROBIT Analysis at 95% hitrate) | 63 | 38 | 23 |
| 95% confidence interval [cp/ml] | 38-161 | 25-82 | 17-39 |

EP 2 402 462 A1

SEQUENCE LISTING

<110> F. Hoffmann-La Roche

<120> Internally controlled multiplex detection and quantification of microbial nucleic acids

<130> 25005 EP-PM

<160> 30

<170> PatentIn version 3.4

<210> 1
<211> 30
<212> DNA
<213> Artificial

<220>
<223> primer/probe

<400> 1
agtgggggga catcaagcag ccatgcaaat 30

<210> 2
<211> 23
<212> DNA
<213> Artificial

<220>
<223> primer/probe

<400> 2
gctttcagcc cagaagtaat acc 23

<210> 3
<211> 25
<212> DNA
<213> Artificial

<220>
<223> primer/probe

<400> 3
ggacacatca agcagccatg caaat 25

<210> 4
<211> 29
<212> DNA
<213> Artificial

<220>
<223> primer/probe

<400> 4
agtgggggga catcaagcag ccatgcaaa 29

<210> 5
<211> 20
<212> DNA
<213> Artificial

<220>
<223> primer/probe

<400> 5
agagaaccaa ggggaagtga 20

<210> 6
<211> 28
<212> DNA
<213> Artificial

<220>
<223> primer/probe

<400> 6
ataatccacc tatcccagta ggagaaat 28

<210> 7
<211> 29
<212> DNA
<213> Artificial

<220>
<223> primer/probe

<400> 7
agtgggggga caccaggcag caatgcaaa 29

<210> 8
<211> 20
<212> DNA
<213> Artificial

<220>
<223> primer/probe

<400> 8
catagcagga actactagta 20

<210> 9
<211> 25
<212> DNA
<213> Artificial

<220>
<223> primer/probe

<400> 9
ctatgtcact tccccttggt tctct 25

<210> 10
<211> 30
<212> DNA
<213> Artificial

<220>
<223> primer/probe

<400> 10
ggtactagta gttcctgcta tatcacttcc 30

<210> 11
<211> 20
<212> DNA
<213> Artificial

<220>
<223> primer/probe

<400> 11
tccttgtctt atgtccagaa 20

<210> 12
<211> 30
<212> DNA
<213> Artificial

<220>
<223> primer/probe

<400> 12
ggtactagta gttcctgcta tgtcacttcc 30

<210> 13
<211> 28
<212> DNA
<213> Artificial

<220>
<223> primer/probe

<400> 13
tttggtcctt gtcttatgtc cagaatgc 28

<210> 14
<211> 28
<212> DNA
<213> Artificial

<220>
<223> primer/probe

<400> 14
tactagtagt tcctgctatg tcacttcc 28

<210> 15
<211> 23
<212> DNA
<213> Artificial

<220>
<223> primer/probe

<400> 15
tgtgttatga tggtgtttaa atc 23

<210> 16
<211> 20
<212> DNA
<213> Artificial

<220>
<223> primer/probe

<400> 16
actctaaagg gttcctttgg 20

<210> 17
<211> 29

<212> DNA
<213> Artificial

<220>
<223> primer/probe

<400> 17
tcagcattat cagaaggagc caccccaca 29

<210> 18
<211> 32
<212> DNA
<213> Artificial

<220>
<223> primer/probe

<400> 18
tctgcagctt cctcattgag gtatctttta ac 32

<210> 19
<211> 41
<212> DNA
<213> Artificial

<220>
<223> primer/probe

<400> 19
atcctgggat taaataaaat agtaagaatg tatagcccta c 41

<210> 20
<211> 29
<212> DNA
<213> Artificial

<220>
<223> primer/probe

<400> 20
accatcaatg agggaagctg cagaatggg 29

<210> 21
<211> 20
<212> DNA
<213> Artificial

<220>
<223> primer/probe

<400> 21
ggctaactag ggacccactg 20

<210> 22
<211> 26
<212> DNA
<213> Artificial

<220>
<223> primer/probe

<400> 22
tgactctggt aactagagat ccctca 26

<210> 23
<211> 18
<212> DNA
<213> Artificial

<220>
<223> primer/probe

<400> 23
actagggaac ccactgct 18

<210> 24
<211> 17
<212> DNA
<213> Artificial

<220>
<223> primer/probe

<400> 24
ggtctgaggg atctcta 17

<210> 25
<211> 24
<212> DNA
<213> Artificial

<220>
<223> primer/probe

<400> 25
ctgctagaga ttttccacac tgac 24

<210> 26
<211> 26
<212> DNA
<213> Artificial

<220>
<223> primer/probe

<400> 26
tcagcaagcc gagtcctgcg tcgaga 26

<210> 27
<211> 27
<212> DNA
<213> Artificial

<220>
<223> primer/probe

<400> 27
ccgctaagcc gagccctttg cgtcgga 27

<210> 28
<211> 34
<212> DNA
<213> Artificial

<220>
<223> primer/probe

```
<400>  28
accagagtca cacaacagac gggcacacac tact                                   34


<210>  29
<211>  28
<212>  DNA
<213>  Artificial

<220>
<223>  primer/probe

<400>  29
tctctagcag tggcgcccga acagggac                                          28


<210>  30
<211>  580
<212>  DNA
<213>  Artificial

<220>
<223>  quantitative standard nucleic acid

<400>  30
tctagatctc agcattatca gaaggagcca ccccacaaga accactaata ctctaatgac      60

aagtgggggg acatcaagca gccatgcaaa tgttaaaaag aaggtgagat gaccagagga     120

ctgagtccaa tatcacgcat agcactatag aactctgcaa gccacaagac aagaagagag     180

aaccaagggg aagtgacata gcaggaacta ctagtacctc ccaaaataag aaacaaataa     240

aagtaatcaa tccggaactt tatcttcaca cctaatggag atgaggatgg taggtgggat     300

taaataaaat agtaagaatg tatagccctg ttgacacttg tacaggcctt tcagcactat     360

taaactgaaa ctagacttct gagagactat actatggaca taaaaaggaa ccaaaataag     420

accaaattgg aaaggacggt tttaaaaaga cggctatgcg agattgccag cgaactaagt     480

gggaagcatc actaacccag cttcagcaat ggtcaggtaa gccggaaatg atgacagcat     540

gtcagggagt gggaaacatg aggattaccc atgtaagctt                             580
```

## Description

### Field of the invention

The present invention relates to new methods and uses for the detection and quantification of microbial nucleic acids employing an internal quantitative reference. Preferred methods are based on the amplification of nucleic acids, preferably the polymerase chain reaction. Further provided are kits comprising components for performing said methods and uses.

### Background of the invention

In the field of molecular diagnostics, the detection and quantification of microbial nucleic acids using nucleic acid amplification reactions plays a significant role. The routine screening of blood donations for the presence of Human Immunodeficiency Virus (HIV), Hepatitis-B (HBV) and/or C Virus (HCV) is an example for the large-scale application of nucleic acid amplification and detection reactions. The latter comprise a variety of different techniques, the most commonly used one being the Polymerase Chain Reaction (PCR) introduced by Kary Mullis in 1984.

Automated systems for PCR-based analysis often make use of real-time detection of product amplification during the PCR process. Key to such methods is the use of modified oligonucleotides carrying reporter groups or labels.

The amplification is performed most commonly with the polymerase chain reaction which specifically amplifies target nucleic acids to detectable amounts. Other possible amplification reactions comprise, among others, the Ligase Chain Reaction, Polymerase Ligase Chain Reaction, Gap-LCR, Repair Chain Reaction, 3SR, NASBA, Strand Displacement Amplification (SDA), Transcription Mediated Amplification (TMA), and Qβ-amplification.

Detection of a microbial nucleic acid in a biological sample is crucial e.g. for recognizing an infection of an individual. Thereby, one important requirement for an assay for detection of a microbial infection is that false-negative results due to variable sequence regions on a microbial genome caused by mutations are to be avoided. For example, individuals infected with HIV are most contagious during the early viremic stages of infection. After onset of the HIV specific immune response plasma viremia declines. The asymptomatic period is characterized by persistent, low level plasma viremia. Mutated or partially mutated sequences within the virus genome that are possibly not amplified and/or detected in combination with the low viral load enhance the possibility of obtaining false-negative results.

Siddappa et al. (J. Clin. Microbiol. 42, (2004), 2742-2751) and Herrmann et al. (J. Clin. Microbiol. 42, (2004), 1909-1914), or US 2004/0229211 use an approach wherein more than one region is amplified by PCR and subsequently detected.

In addition to mere detection of the presence or absence of a microbial nucleic acid in a sample, it is often important to determine the quantity of said nucleic acid. Stage and severity of a viral disease may be assessed on the basis of the viral load. Further, monitoring of any therapy requires information on the quantity of a virus present in an individual in order to evaluate the therapy's success. The references mentioned above, dealing with the detection of microbial nucleic acids by simultaneously targeting multiple sequence portions of the respective microorganism, all use external calibration for quantifying the presence of said nucleic acids, i.e. standard curves are created in separate reactions using known amounts of identical or comparable nucleic acids. The absolute quantity of a microbial nucleic acid is subsequently determined by comparison of the result obtained with the analyzed sample with said standard function. External calibration, however, has the disadvantage that a possible extraction procedure, its varied efficacy, and the possible and often not predictable presence of agents inhibiting the amplification and/or detection reaction are not taken into consideration. This circumstance also applies to any other sample-related effects. Therefore, it might be the case that a sample is judged as negative due to an unsuccessful extraction procedure or other sample-based factors, whereas the microbial nucleic acid to be detected and quantified is actually present in the sample.

Thus, there is a need in the art to provide a method for the simple and reliable detection and quantification of a microbial nucleic acid.

### Description of the invention

The present invention relates to new methods and uses for the detection and quantification of microbial nucleic acids employing one or more internal quantitative references. In brief, multiple sequence portions of a microbial nucleic acid are simultaneously amplified and detected along with said internal quantitative reference in the same reaction mixture, allowing quantification of the microbial nucleic acid. Thus, one subject of the present invention is:

A method for detecting and quantifying a microbial nucleic acid in a biological sample, said method comprising:

a) providing a reaction mixture comprising one or more quantitative standard nucleic acids, two or more primer pairs, said primer pairs being specific for different sequence portions of said microbial nucleic acid, and one or more primer pairs specific for said one or more standard nucleic acids

b) adding said biological sample to said reaction mixture

c) performing one or more cycling steps, wherein a cycling step comprises an amplifying step, said amplifying step comprising producing two or more different amplification products derived from said microbial nucleic acid if present in said sample and producing one or more amplification products derived from said one or more quantitative standard nucleic acids

d) detecting and measuring detectable signals generated by said amplification products and proportional to their concentration in said reaction mixture, wherein the presence or absence of the detectable signal generated by said microbial nucleic acid is indicative of the presence or absence of the microbial nucleic acid in said biological sample

e) determining the quantity of said microbial nucleic acid in said biological sample by comparison of the signals generated by said microbial nucleic acid and said one or more quantitative standard nucleic acids, wherein said two or more different sequence portions of the microbial nucleic acid are sequence portions of the same microorganism.

The method according to the invention adds a number of improvements to the art:

The exploitation of more than one sequence portion of a microbial nucleic acid leads to a significantly reduced risk to underquantitate microbial titers, and at the same time the risk to underestimate the titers of unknown microbial isolates is reduced. All different genotypes within any given organism can be readily detected and quantified using a minimum number of oligonucleotides as a result of the decreased sensitivity to amplification efficiency variability.

The life cycle of tests formulated according to the invention is prolonged, since it is able to deal even with new variants generated by microorganisms such as viruses through selective pressure for example as a consequence of new anti- retro viral drugs.

Overall sensitivity is significantly increased, and the variability of titer determinations is minimized due to the simultaneous detection and quantification of multiple different sequence portions.

By using one or more "internal" quantitative standard nucleic acids according to the method or methods of the invention, sample-specific, but also sample-unspecific inhibitory effects possibly interfering with the amplification and detection reactions for quantitative standard nucleic acid and microbial nucleic acid simultaneously (target region-independent inhibition) are leveled resulting in more accurate titers. "Internal" means that the one or more quantitative standard nucleic acids are amplified, detected and quantified within the same reaction mixture as the microbial nucleic acid instead of in a separate experiment.

Failure or reduction of amplification efficiency specific for one target sequence portion but not affecting said one or more quantitative standard nucleic acids may result in incorrect (underestimation of) titers (e.g. in the case of mutations in the target nucleic acid at the 3' end of the primers, target specific secondary structure e.g. for HCV genotypes). By including one or more further target sequence portions, the probability that both targets show decreased/disrupted amplification efficiency is reduced and therefore the chance of generating an incorrect titer is considerably decreased.

The quantitative standard nucleic acid or acids in quantitative tests have a rather high concentration so that they are still amplified and detected in samples with a high concentration of target nucleic acid. Therefore, the monitoring of low positive samples at the limit of detection (LOD) is not stringent, especially if the amplification reaction is partly suppressed. By using multiple different sequence portions for detection, the LOD can be ensured because the probability that two or more different target sequence portions are suppressed is reduced.

In a preferred embodiment, the invention provides the method described above, additionally comprising:

in step a) providing two or more probes specific for the different sequence portions amplified by said two or more primer pairs specific for different sequence portions of said microbial nucleic acid, and one or more probes specific for the sequence portion or portions amplified by said one or more primer pairs specific for said one or more quantitative standard nucleic acids.

Conventional techniques of molecular biology and nucleic acid chemistry, which are within the skill of the art, are explained in the literature. See, for example, Sambrook J. et al., Molecular Cloning: A Laboratory Manual, Cold Spring Harbor Laboratory Press, Cold Spring Harbor, New York, 1989, Gait, M.J., ed., 1984; Nucleic Acid Hybridization, Hames, B.D., and Higgins, S.J., eds., 1984; and a series, Methods in Enzymology, Academic Press, Inc.

A "reaction mixture" as used in the present invention comprises at least all components to facilitate a biological or chemical reaction. It is a single volume without any separating compartments, i.e. all components present in said "reaction mixture" are in immediate contact with each other.

A "biological sample" can be any sample of natural origin. Preferably, a "biological sample" is derived from a human and is a body liquid. In a preferred embodiment of the invention, the "biological sample" is blood.

As is known in the art, a "nucleoside" is a base-sugar combination. The base portion of the nucleoside is normally a heterocyclic base. The two most common classes of such heterocyclic bases are the purines and the pyrimidines.

"Nucleotides" are "nucleosides" that further include a phosphate group covalently linked to the sugar portion of the nucleoside. For those "nucleosides" that include a pentofuranosyl sugar, the phosphate group can be linked to either the 2', 3' or 5' hydroxyl moiety of the sugar. A "nucleotide" is the "monomeric unit" of an "oligonucleotide", more generally denoted herein as an "oligomeric compound", or a "polynucleotide", more generally denoted as a "polymeric compound". Another general expression for the aforementioned is deoxyribonucleic acid (DNA) and ribonucleic acid (RNA).

According to the invention, an "oligomeric compound" is a compound consisting of "monomeric units" which may be "nucleotides" alone or "non-natural compounds" (see below), more specifically "modified nucleotides" (or "nucleotide analogs") or "non-nucleotide compounds", alone or combinations thereof. "Oligonucleotides" and "modified oligonucleotides" (or "oligonucleotide analogs") are subgroups of "oligomeric compounds" in the context of the invention.

In the context of this invention, the term "oligonucleotide" refers to "polynucleotides" formed from a plurality of "nucleotides" as the "monomeric unit", i.e. an "oligonucleotide" belongs to a specific subgroup of a "oligomeric compound" or "polymeric compound" of ribonucleic acid (RNA) or deoxyribonucleic acid (DNA) with "monomeric units". The phosphate groups are commonly referred to as forming the internucleoside backbone of the "oligonucleotide". The normal linkage or backbone of RNA and DNA is a 3' to 5' phosphodiester linkage.

"Oligonucleotides" and "modified oligonucleotides" (see below) according to the invention may be synthesized as principally described in the art and known to the expert in the field. Methods for preparing oligomeric compounds of specific sequences are known in the art, and include, for example, cloning and restriction of appropriate sequences and direct chemical synthesis. Chemical synthesis methods may include, for example, the phosphotriester method described by Narang S. A. et al., Methods in Enzymology 68 (1979) 90-98, the phosphodiester method disclosed by Brown E. L., et al., Methods in Enzymology 68 (1979) 109-151, the phosphoramidite method disclosed in Beaucage et al., Tetrahedron Letters 22 (1981) 1859, the H-phosphonate method disclosed in Garegg et al., Chem. Scr. 25 (1985) 280-282 and the solid support method disclosed in US 4,458,066.

For the above-described method, the nucleic acids can be present in double-stranded or single-stranded form whereby the double-stranded nucleic acids are denatured, i.e. made single-stranded, before the method is performed by heating, i.e. thermal denaturing.

In another preferred embodiment, a primer and/ or the probe may be chemically modified, i.e. the primer and/ or the probe comprise a modified nucleotide or a non-nucleotide compound. The probe or the primer is then a modified oligonucleotide.

"Modified nucleotides" (or "nucleotide analogs") differ from a natural "nucleotide" by some modification but still consist of a base, a pentofuranosyl sugar, a phosphate portion, base-like, pentofuranosyl sugar-like and phosphate-like portion or combinations thereof. For example, a "label" may be attached to the base portion of a "nucleotide" whereby a "modified nucleotide" is obtained. A natural base in a "nucleotide" may also be replaced by e.g. a 7-deazapurine whereby a "modified nucleotide" is obtained as well. The terms "modified nucleotide" or "nucleotide analog" are used interchangeably in the present application. A "modified nucleoside" (or "nucleoside analog") differs from a natural nucleoside by some modification in the manner as outlined above for a "modified nucleotide" (or a "nucleotide analog").

A "non-nucleotide compound" is different from a natural "nucleotide" but is in the sense of this invention still capable - similar to a "nucleotide" - of being a "monomeric unit" of an "oligomeric compound". Therefore, a "non-nucleotide compound" has to be capable of forming an "oligomeric compound" with "nucleotides". Even "non-nucleotide compounds" may contain a base-like, pentofuranosyl sugar-like or a phosphate-like portions, however, not all of them are present at the same time in a "non-nucleotide compound".

A "modified oligonucleotide" (or "oligonucleotide analog") belongs to another specific subgroup of the "oligomeric compounds", that possesses one or more "nucleotides", one or more "non-nucleotide compounds" or "modified nucleotides" as "monomeric units". Thus, the terms "modified oligonucleotide" (or "oligonucleotide analog") refers to structures that function in a manner substantially similar to "oligonucleotides" and are used interchangeably throughout the application. From a synthetical point of view, a "modified oligonucleotide" (or a "oligonucleotide analog") can be for example made by chemical modification of "oligonucleotides" by appropriate modification of the phosphate backbone, ribose unit or the nucleotide bases (Uhlmann and Peyman, Chemical Reviews 90 (1990) 543; Verma S., and Eckstein F., Annu. Rev. Biochem. 67 (1998) 99-134). Representative modifications include phosphorothioate, phosphorodithioate, methyl phosphonate, phosphotriester or phosphoramidate inter-nucleoside linkages in place of phosphodiester inter-nucleoside linkages; deaza- or aza-purines and -pyrimidines in place of natural purine and pyrimidine bases, pyrimidine bases having substituent groups at the 5 or 6 position; purine bases having altered substituent groups at the 2, 6 or 8 positions or 7 position as 7-deazapurines; bases carrying alkyl-, alkenyl-, alkinyl or aryl-moieties, e.g. lower alkyl groups such as methyl, ethyl, propyl, butyl, tert-butyl, pentyl, hexyl, heptyl, octyl, nonyl, decyl, or aryl groups like phenyl, benzyl, naphtyl; sugars having substituent groups at, for example, their 2' position; or carbocyclic or acyclic sugar analogs. Other modifications consistent with the spirit of this invention are known to those skilled in the art. Such "modified oligonucleotides" (or "oligonucleotide analogs") are best described as being functionally interchangeable with, yet structurally different from, natural "oligonucleotides" (or synthetic "oligonucleotides" along natural lines). In more detail, exemplary modifications are disclosed in Verma S., and Eckstein F., Annu. Rev. Biochem. 67 (1998) 99-134 or WO 02/12263. In addition, modification can be made wherein nucleoside units are joined through groups that substitute for the internucleoside phosphate or sugar phosphate linkages. Such linkages include those disclosed in Verma S., and Eckstein F., Annu. Rev. Biochem. 67 (1998) 99-134. When other than phosphate linkages are utilized to link the nucleoside units, such structures have also been described as "oligonucleosides".

A "nucleic acid" as well as the "target nucleic acid" or the "microbial nucleic acid" is a polymeric compound of "nucleotides" as known to the expert skilled in the art. "Target nucleic acid" or "microbial nucleic acid" is used herein to denote a "nucleic acid" in a sample which should be analyzed, i.e. the presence, non-presence or amount thereof in a sample should be determined. Therefore, in this case the nucleic acid is the target and can therefore be also denoted as "target nucleic acid". Since, according to the invention, the target nucleic acid is of microbial origin, the target nucleic acid is also referred to as "microbial nucleic acid". For example, if it has to be determined whether blood contains HIV, the "target nucleic acid" or "microbial nucleic acid" is the nucleic acid of HIV.

"Microorganism" means any virus, bacterium, archaeum, fungus or any unicellular eukaryotic organism.

"Microbial" means derived from or belonging to a "microorganism".

A "quantitative standard nucleic acid" is a "nucleic acid" and thus a polymeric compound of "nucleotides" as known to the expert skilled in the art. In the case of the "quantitative standard nucleic acid", the nucleic acid is apt to be and used as a reference in order to determine the quantity of the "target nucleic acid" or "microbial nucleic acid". For this purpose, the "quantitative standard nucleic acid" undergoes all possible sample preparation steps along with the "target nucleic acid" or the "microbial nucleic acid". Moreover, it is processed throughout the method within the same reaction mixture. The "quantitative standard nucleic acid" must generate, directly or indirectly, a detectable signal both in the presence or absence of the target nucleic acid. For this purpose, the concentration of the "quantitative standard nucleic acid" has to be carefully optimized in each test in order not to interfere with sensitivity but in order to generate a detectable signal also e.g. at very high target concentrations. Preferably, the concentration range for the "quantitative standard nucleic acid" will comprise a range of 100 copies per reaction to 100 000 copies per reaction (e.g. HIV: 1000 copies / reaction, HCV: 7500 copies/reaction). The final concentration of the QS in the reaction mixture is dependent on the quantitative measuring range accomplished. The "quantitative standard nucleic acid " can be, for example, DNA, RNA or PNA, armored DNA or RNA and modified forms thereof.

The term "primer" is used herein as known to the expert skilled in the art and refers to "oligomeric compounds", primarily to "oligonucleotides", but also to "modified oligonucleotides" that are able to "prime" DNA synthesis by a template-dependent DNA polymerase, i.e. the 3'-end of the e.g. oligonucleotide provides a free 3'-OH group whereto further "nucleotides" may be attached by a template-dependent DNA polymerase establishing 3' to 5' phosphodiester linkage whereby deoxynucleoside triphosphates are used and whereby pyrophosphate is released. Therefore, there is - except for the intended function - no fundamental difference between a "primer", an "oligonucleotide" or a "probe" as used in the present invention.

For the above-described method, the nucleic acids can be present in double-stranded or single-stranded form whereby the double-stranded nucleic acids are denatured, i.e. made single-stranded, before the method is performed by heating, i.e. thermal denaturing.

In another preferred embodiment, a primer and/ or the probe may be chemically modified, i.e. the primer and/ or the probe comprise a modified nucleotide or a non-nucleotide compound. The probe or the primer is then a modified oligonucleotide.

"Labels", often referred to as "reporter groups", are generally groups that make a nucleic acid, in particular the "oligomeric compound" or the "modified oligonucleotide", as well as any nucleic acids bound thereto distinguishable from the remainder of the sample (nucleic acids having attached a "label" can also be termed labeled nucleic acid binding compounds, labeled probes or just probes). Preferred labels according to the invention are fluorescent labels, which are e.g. "fluorescent dyes" as a fluorescein dye, a rhodamine dye, a cyanine dye, and a coumarin dye. Preferred "fluorescent dyes" according to the invention are FAM, HEX, CY5, JA270, Cyan, CY5.5, LC-Red 640, LC-Red 705.

A "detectable signal" is a signal "generated", by a compound such as the "microbial nucleic acid" or the "quantitative standard nucleic acid", rendering said compound distinguishable from the remainder of the sample. According to the invention, said "detectable signal" can be quantified. A "detectable signal" can be e.g. radioactive or optical such as luminescent signals. Preferred "detectable signals" according to the invention are fluorescent signals emitted by "fluorescent dyes".

"To generate" means to produce, directly or indirectly. In the context of a "detectable signal", "to generate" can therefore mean "to produce directly", e.g. in the case of a fluorescent dye emitting a fluorescent signal, or "to produce indirectly" in the sense of "to evoke" or "to induce", such as a "microbial nucleic acid" "generating" a "detectable signal" via a "label" such as a "fluorescent dye", or via a nucleic acid probe carrying a "label" such as a "fluorescent dye".

As known by the person skilled in the art, the term "specific" in the context of primers and probes implies that a primer or probe "specific" for a distinct nucleic acid binds to said nucleic acid under stringent conditions. Preferably, the primers and probes used in the method according to the invention are at least 80% identical to sequence portions of the microbial nucleic acid and/or the quantitative standard nucleic acid or acids. In a more preferred embodiment of the invention, the primer sequences are at least 12 contiguous nucleotides selected from the group of SEQ ID NO 1-16, 21-27, and the probes are at least 12 contiguous nucleotides selected from the group of SEQ ID NO 17-20, 28, 29 or the corresponding complementary nucleic acid sequences thereof. More preferably, the selected sequences consist of 12 to 60 nucleotides, yet more preferably of 20 to 60 nucleotides, most preferably of the exact sequences selected from said groups of sequences or their complementary nucleic acid sequences.

The "Polymerase Chain Reaction" (PCR) is disclosed, among other references, in U.S. Patent Nos. 4,683,202, 4,683,195, 4,800,159, and 4,965,188 and is the most preferred nucleic acid amplification technique used for method according to the invention. PCR typically employs two or more oligonucleotide primers that bind to a selected nucleic acid template (e.g. DNA or RNA). Primers useful in the present invention include oligonucleotides capable of acting as a point of initiation of nucleic acid synthesis within the nucleic acid sequences of the microbial nucleic acid or quantitative standard nucleic acid. A primer can be purified from a restriction digest by conventional methods, or it can be produced synthetically. The primer is preferably single-stranded for maximum efficiency in amplification, but the primer can be double-stranded. Double-stranded primers are first denatured, i.e., treated to separate the strands. One method of denaturing double stranded nucleic acids is by heating. A "thermostable polymerase" is a polymerase enzyme that is heat stable, i.e., it is an enzyme that catalyzes the formation of primer extension products complementary to a template and does not irreversibly denature when subjected to the elevated temperatures for the time necessary to effect denaturation of double-stranded template nucleic acids. Generally, the synthesis is initiated at the 3' end of each primer and proceeds in the 5' to 3' direction along the template strand. Thermostable polymerases have been isolated from Thermus flavus, T. ruber, T. thermophilus, T. aquaticus, T. lacteus, T. rubens, Bacillus stearothermophilus, and Methanothermus fervidus. Nonetheless, polymerases that are not thermostable also can be employed in PCR assays provided the enzyme is replenished. If the template nucleic acid is double-stranded, it is necessary to separate the two strands before it can be used as a template in PCR. Strand separation can be accomplished by any suitable denaturing method including physical, chemical or enzymatic means. One method of separating the nucleic acid strands involves heating the nucleic acid until it is predominately denatured (e.g., greater than 50%, 60%, 70%, 80%, 90% or 95% denatured). The heating conditions necessary for denaturing template nucleic acid will depend, e.g., on the buffer salt concentration and the length and nucleotide composition of the nucleic acids being denatured, but typically range from about 90°C to about 105°C for a time depending on features of the reaction such as temperature and the nucleic acid length. Denaturation is typically performed for about 30 sec to 4 min (e.g., 1 min to 2 min 30 sec, or 1.5 min). If the double-stranded template nucleic acid is denatured by heat, the reaction mixture is allowed to cool to a temperature that promotes annealing of each primer to its target sequence on the microbial nucleic acid and/or quantitative standard nucleic acid. The temperature for annealing is usually from about 35°C to about 65°C (e.g., about 40°C to about 60°C; about 45°C to about 50°C). Annealing times can be from about 10 sec to about 1 min (e.g., about 20 sec to about 50 sec; about 30 sec to about 40 sec). The reaction mixture is then adjusted to a temperature at which the activity of the polymerase is promoted or optimized, i.e., a temperature sufficient for extension to occur from the annealed primer to generate products complementary to the microbial nucleic acid and/or quantitative standard nucleic acid. The temperature should be sufficient to synthesize an extension product from each primer that is annealed to a nucleic acid template, but should not be so high as to denature an extension product from its complementary template (e.g., the temperature for extension generally ranges from about 40° to 80°C (e.g., about 50°C to about 70°C; about 60°C). Extension times can be from about 10 sec to about 5 min (e.g., about 30 sec to about 4 min; about 1 min to about 3 min; about 1 min 30 sec to about 2 min). The newly synthesized strands form a double-stranded molecule that can be used in the succeeding steps of the reaction. The steps of strand separation, annealing, and elongation can be repeated as often as needed to produce the desired quantity of amplification products corresponding to the microbial nucleic acid and/or quantitative standard nucleic acid. The limiting factors in the reaction are the amounts of primers, thermostable enzyme, and nucleoside triphosphates present in the reaction. The cycling steps (i.e., denaturation, annealing, and extension) are preferably repeated at least once. For use in detection, the number of cycling steps will depend, e.g., on the nature of the sample. If the sample is a complex mixture of nucleic acids, more cycling steps will be required to amplify the target sequence sufficient for detection. Generally, the cycling steps are repeated at least about 20 times, but may be repeated as many as 40, 60, or even 100 times.

"Limit of detection" or "LOD" means the lowest detectable amount or concentration of a nucleic acid in a sample. A low "LOD" correspond to high sensitivity and vice versa. The "LOD" is usually expressed by means of the unit "cp/ml", particularly if the nucleic acid is a viral nucleic acid. "Cp/ml" means "copies per milliliter" wherein a "copy" is copy of the respective nucleic acid.

A widely used method for calculating an LOD is "Probit Analysis", which is a method of analyzing the relationship between a stimulus (dose) and the quantal (all or nothing) response. In a typical quantal response experiment, groups of animals are given different doses of a drug. The percent dying at each dose level is recorded. These data may then be analyzed using Probit Analysis. The Probit Model assumes that the percent response is related to the log dose as the cumulative normal distribution. That is, the log doses may be used as variables to read the percent dying from the cumulative normal. Using the normal distribution, rather than other probability distributions, influences the predicted response rate at the high and low ends of possible doses, but has little influence near the middle.

The "Probit Analysis" can be applied at distinct "hitrates". As known in the art, "hitrate" is commonly expressed in percent [%] and indicates the percentage of positive results at a specific concentration of an analyte. Thus for example, an LOD can be determined at 95% hitrate, which means that the LOD is calculated for a setting in which 95% of the valid results are positive.

Nucleic acid amplification reactions apart from PCR comprise the Ligase Chain Reaction (LCR; Wu D. Y. and Wallace R. B., Genomics 4 (1989) 560-69; and Barany F., Proc. Natl. Acad. Sci. USA 88 (1991)189-193); Polymerase Ligase Chain Reaction (Barany F., PCR Methods and Applic. 1 (1991) 5-16); Gap-LCR (WO 90/01069); Repair Chain Reaction (EP 0439182 A2), 3SR (Kwoh D. Y. et al., Proc. Natl. Acad. Sci. USA 86 (1989) 1173-1177; Guatelli J.C., et al., Proc. Natl. Acad. Sci. USA 87 (1990) 1874-1878; WO 92/08808), and NASBA (US 5,130,238). Further, there are strand displacement amplification (SDA), transcription mediated amplification (TMA), and Qβ-amplification (for a review see e.g. Whelen A. C. and Persing D. H., Annu. Rev. Microbiol. 50 (1996) 349-373; Abramson R. D. and Myers T. W., Curr Opin Biotechnol 4 (1993) 41-47).

Suitable nucleic acid detection methods are known to the expert in the field and are described in standard textbooks as Sambrook J. et al., Molecular Cloning: A Laboratory Manual, Cold Spring Harbor Laboratory Press, Cold Spring Harbor, New York, 1989 and Ausubel F. et al.: Current Protocols in Molecular Biology 1987, J. Wiley and Sons, NY. There may be also further purification steps before the nucleic acid detection step is carried out as e.g. a precipitation step. The detection methods may include but are not limited to the binding or intercalating of specific dyes as ethidium bromide which intercalates into the double-stranded DNA and changes its fluorescence thereafter. The purified nucleic acid may also be separated by electrophoretic methods optionally after a restriction digest and visualized thereafter. There are also probe-based assays which exploit the oligonucleotide hybridization to specific sequences and subsequent detection of the hybrid. It is also possible to sequence the nucleic acid after further steps known to the expert in the field. Preferred template-dependent nucleic acid polymerase is the ZO5 DNA polymerase and mutations thereof. Other template-dependent nucleic acid polymerases useful in the invention are Taq polymerase and Tth Polymerase. Yet other nucleic acid polymerases useful for the methods according to the invention are known to the skilled artisan.

Before nucleic acids may be analyzed in one of the above-mentioned assays, they have to be isolated or purified from biological samples containing complex mixtures of different components. Often, for the first steps, processes are used which allow the enrichment of the nucleic acids. To release the contents of cells or viral particles, they may be treated with enzymes or with chemicals to dissolve, degrade or denature the cellular walls or viral particles. This process is commonly referred to as lysis. The resulting solution containing such lysed material is referred to as lysate. A problem often encountered during lysis is that other enzymes degrading the component of interest, e.g. deoxyribonucleases or ribonucleases degrading nucleic acids, come into contact with the component of interest during the lysis procedure. These degrading enzymes may also be present outside the cells or may have been spatially separated in different cellular compartments prior to lysis. As the lysis takes place, the component of interest becomes exposed to said degrading enzymes. Other components released during this process may e.g. be endotoxins belonging to the family of lipopolysaccharides which are toxic to cells and can cause problems for products intended to be used in human or animal therapy.

There is a variety of means to tackle the above-mentioned problem. It is common to use chaotropic agents such as guanidinium thiocyanate or anionic, cationic, zwitterionic or non-ionic detergents when nucleic acids are intended to be set free. It is also an advantage to use proteases which rapidly degrade the previously described enzymes or unwanted proteins. However, this may produce another problem as said substances or enzymes can interfere with reagents or components in subsequent steps.

Enzymes which can be advantageously used in such lysis or sample preparation processes mentioned above are enzymes which cleave the amide linkages in protein substrates and which are classified as proteases, or (interchangeably) peptidases (see Walsh, 1979, Enzymatic Reaction Mechanisms. W. H. Freeman and Company, San Francisco, Chapter 3). Proteases used in the prior art comprise alkaline proteases (WO 98/04730) or acid proteases (US 5,386,024). A protease which has been widely used for sample preparation in the isolation of nucleic acids in the prior art is proteinase K from Tritirachium album (see e.g. Sambrook J. et al., Molecular Cloning: A Laboratory Manual, Cold Spring Harbor Laboratory Press, Cold Spring Harbor, New York, 1989) which is active around neutral pH and belongs to a family of proteases known to the person skilled in the art as subtilisins. Especially advantageous for the use in lysis or sample preparation processes mentioned above is the enzyme esperase, a robust protease that retains its activity at both high alkalinity and at high temperatures (EP 1 201 753).

In the sample preparation steps following the lysis step, the component of interest is further enriched. If the non-proteinaceous components of interest are e.g. nucleic acids, they are normally extracted from the complex lysis mixtures before they are used in a probe-based assay.

There are several methods for the extraction of nucleic acids:

- sequence-dependent or biospecific methods as e.g.:
  - affinity chromatography
  - hybridization to immobilized probes
- sequence-independent or physico-chemical methods as e.g.:
  - liquid-liquid extraction with e.g. phenol-chloroform
  - precipitation with e.g. pure ethanol
  - extraction with filter paper
  - extraction with micelle-forming agents as cetyl-trimethyl-ammonium-bromide
  - binding to immobilized, intercalating dyes, e.g. acridine derivatives
  - adsorption to silica gel or diatomic earths
  - adsorption to magnetic glass particles (MGP) or organo-silane particles under chaotropic conditions

Particularly interesting for extraction purposes is the adsorption of nucleic acids to a glass surface although other surfaces are possible. Many procedures for isolating nucleic acids from their natural environment have been proposed in recent years by the use of their binding behavior to glass surfaces. If unmodified nucleic acids are the target, a direct binding of the nucleic acids to a material with a silica surface is preferred because, among other reasons, the nucleic acids do not have to be modified, and even native nucleic acids can be bound. These processes are described in detail by various documents. In Vogelstein B. et al., Proc. Natl. Acad. USA 76 (1979) 615-9, for instance, a procedure for binding nucleic acids from agarose gels in the presence of sodium iodide to ground flint glass is proposed. The purification of plasmid DNA from bacteria on glass dust in the presence of sodium perchlorate is described in Marko M. A. et al., Anal. Biochem. 121 (1982) 382-387. In DE-A 37 34 442, the isolation of single-stranded M13 phage DNA on glass fiber filters by precipitating phage particles using acetic acid and lysis of the phage particles with perchlorate is described. The nucleic acids bound to the glass fiber filters are washed and then eluted with a methanol-containing Tris/EDTA buffer. A similar procedure for purifying DNA from lambda phages is described in Jakobi R. et al., Anal. Biochem. 175 (1988) 196-201. The procedure entails the selective binding of nucleic acids to glass surfaces in chaotropic salt solutions and separating the nucleic acids from contaminants such as agarose, proteins or cell residue. To separate the glass particles from the contaminants, the particles may be either centrifuged or fluids are drawn through glass fiber filters. This is a limiting step, however, that prevents the procedure from being used to process large quantities of samples. The use of magnetic particles to immobilize nucleic acids after precipitation by adding salt and ethanol is more advantageous and described e.g. in Alderton R. P. et al., S., Anal. Biochem. 201 (1992) 166-169 and PCT GB 91/00212. In this procedure, the nucleic acids are agglutinated along with the magnetic particles. The agglutinate is separated from the original solvent by applying a magnetic field and performing a wash step. After one wash step, the nucleic acids are dissolved in a Tris buffer. This procedure has a disadvantage, however, in that the precipitation is not selective for nucleic acids. Rather, a variety of solid and dissolved substances are agglutinated as well. As a result, this procedure can not be used to remove significant quantities of any inhibitors of specific enzymatic reactions that may be present. Magnetic, porous glass is also available on the market that contains magnetic particles in a porous, particular glass matrix and is covered with a layer containing streptavidin. This product can be used to isolate biological materials, e.g., proteins or nucleic acids, if they are modified in a complex preparation step so that they bind covalently to biotin. Magnetizable particular adsorbents proved to be very efficient and suitable for automatic sample preparation. Ferrimagnetic and ferromagnetic as well as superparamagnetic pigments are used for this purpose. The most preferred MGPs and methods using magnetic glass particles are those described in WO 01/37291. Particularly useful for the nucleic acid isolation in the context of the invention is the method according to R. Boom et al. (J Clin Microbiol. 28 (1990), 495-503). After the purification or isolation of the nucleic acids including the target nucleic acid from their natural surroundings, the target nucleic acid may be detected.

In an embodiment, the method of the invention includes steps to avoid contamination. For example, an enzymatic method utilizing uracil-DNA glycosylase is described in U.S. Patent Nos. 5,035,996, 5,683,896 and 5,945,313 to reduce or eliminate contamination between one thermocycler run and the next. In addition, standard laboratory containment practices and procedures are desirable when performing the method of the invention. Containment practices and procedures include, but are not limited to, separate work areas for different steps of a method, containment hoods, barrier filter pipette tips and dedicated air displacement pipettes. Consistent containment practices and procedures by personnel are necessary for accuracy in a diagnostic laboratory handling clinical samples.

The methods set out above are preferably based on Fluorescence Resonance Energy Transfer (FRET) between a donor fluorescent moiety and an acceptor fluorescent moiety. A representative donor fluorescent moiety is fluorescein, and representative corresponding acceptor fluorescent moieties include LC-Red 640, LC-Red 705, Cy5, and Cy5.5. Typically, the detecting step includes exciting the sample at a wavelength absorbed by the donor fluorescent moiety and visualizing and/or measuring the wavelength emitted by the corresponding acceptor fluorescent moiety. According to the invention, the detection is followed by quantitating the FRET. Preferably, the detecting step is performed after each cycling step. Most preferably, the detecting step is performed in real time. By using commercially available real-time PCR instrumentation (e.g., LightCycler™ or TaqMan® ), PCR amplification and detection of the amplification product can be combined in a single closed cuvette with dramatically reduced cycling time. Since detection occurs concurrently with amplification, the real-time PCR methods obviate the need for manipulation of the amplification product, and diminish the risk of cross-contamination between amplification products. Real-time PCR greatly reduces turn-around time and is an attractive alternative to conventional PCR techniques in the clinical laboratory.

The following patent applications describe real-time PCR as used in the LightCycler™ technology: WO 97/46707, WO 97/46714 and WO 97/46712. The LightCycler™ instrument is a rapid thermal cycler combined with a microvolume fluorometer utilizing high quality optics. This rapid thermocycling technique uses thin glass cuvettes as reaction vessels. Heating and cooling of the reaction chamber are controlled by alternating heated and ambient air. Due to the low mass of air and the high ratio of surface area to volume of the cuvettes, very rapid temperature exchange rates can be achieved within the thermal chamber.

TaqMan® technology utilizes a single-stranded hybridization probe labeled with two fluorescent moieties. When a first fluorescent moiety is excited with light of a suitable wavelength, the absorbed energy is transferred to a second fluorescent moiety according to the principles of FRET. The second fluorescent moiety is generally a quencher molecule. Typical fluorescent dyes used in this format are for example, among others, FAM, HEX, CY5, JA270, Cyan and CY5.5. During the annealing step of the PCR reaction, the labeled hybridization probe binds to the target nucleic acid (i.e., the amplification product) and is degraded by the 5' to 3' exonuclease activity of the Taq or another suitable polymerase as known by the skilled artisan, such as the preferred ZO5 polymerase, during the subsequent elongation phase. As a result, the excited fluorescent moiety and the quencher moiety become spatially separated from one another. As a consequence, upon excitation of the first fluorescent moiety in the absence of the quencher, the fluorescence emission from the first fluorescent moiety can be detected.

In both detection formats described above, the intensity of the emitted signal can be correlated with the number of original target nucleic acid molecules.

As an alternative to FRET, an amplification product can be detected using a double-stranded DNA binding dye such as a fluorescent DNA binding dye (e.g., SYBRGREEN I® or SYBRGOLD® (Molecular Probes)). Upon interaction with the double-stranded nucleic acid, such fluorescent DNA binding dyes emit a fluorescence signal after excitation with light at a suitable wavelength. A double-stranded DNA binding dye such as a nucleic acid intercalating dye also can be used. When double-stranded DNA binding dyes are used, a melting curve analysis is usually performed for confirmation of the presence of the amplification product.

Therefore, in a preferred embodiment of the invention, the method described above comprising providing several probes additionally comprises

in step c) a hybridizing step comprised by said cycling step, wherein said hybridizing step comprises hybridizing the different sequence portions amplified by said two or more primer pairs specific for different sequence portions of said microbial nucleic acid, if the microbial nucleic acid is present in said sample, with said two or more probes, and hybridizing said sequence portion or portions amplified by said one or more primer pairs specific for said one or more quantitative standard nucleic acids with one or more probes, wherein the probes are labeled with a donor fluorescent moiety and a corresponding acceptor fluorescent moiety

in step d) detecting the presence or absence of fluorescence resonance energy transfer (FRET) between said donor fluorescent moiety and said acceptor fluorescent moiety of said probes, wherein the presence or absence of fluorescence generated by said two or more probes hybridizing to the different sequence portions amplified by said two or more primer pairs specific for different sequence portions of said microbial nucleic acid is indicative of the presence or absence of the microbial nucleic acid in said biological sample

in step e) determining the quantity of said microbial nucleic acid in said sample by comparison of the fluorescent signals generated by said two or more probes hybridizing to the different sequence portions amplified by said two or more primer pairs specific for different sequence portions of said microbial nucleic acid and said one or more probes hybridizing to said sequence portion or portions amplified by said one or more primer pairs specific for said one or more quantitative standard nucleic acids.

The circumstance that said one or more quantitative standard nucleic acids are processed in the same reaction mixture as the microbial nucleic acid suspected to be present in the biological sample provides the advantage, among others, that sample-related inhibitory effects equally affect the amplification and detection reactions of the microbial nucleic acid and said one or more quantitative standard nucleic acids, thus quantification can be performed more accurately.

Molecular beacons in conjunction with FRET can also be used to detect the presence of an amplification product using the real-time PCR methods of the invention. Molecular beacon technology uses a hybridization probe labeled with a first fluorescent moiety and a second fluorescent moiety. The second fluorescent moiety is generally a quencher, and the fluorescent labels are typically located at each end of the probe. Molecular beacon technology uses a probe oligonucleotide having sequences that permit secondary structure formation (e.g., a hairpin). As a result of secondary structure formation within the probe, both fluorescent moieties are in spatial proximity when the probe is in solution. After hybridization to the amplification products, the secondary structure of the probe is disrupted and the fluorescent moieties become separated from one another such that after excitation with light of a suitable wavelength, the emission of the first fluorescent moiety can be detected.

Thus, in a preferred method according to the invention is the method described above using FRET, wherein said probes comprise a nucleic acid sequence that permits secondary structure formation, wherein said secondary structure formation results in spatial proximity between said first and second fluorescent moiety.

Efficient FRET can only take place when the fluorescent moieties are in direct local proximity and when the emission spectrum of the donor fluorescent moiety overlaps with the absorption spectrum of the acceptor fluorescent moiety.

Thus, in a preferred embodiment of the invention, said donor and acceptor fluorescent moieties are within no more than 5 nucleotides of each other on said probe.

In a further preferred embodiment, said acceptor fluorescent moiety is a quencher.

In order be able to render the method according to the invention independent from sequence-specific effects in connection with the different sequence portions of the microbial nucleic acid detected and/or quantified in the present invention, in a preferred embodiment the method according to the invention comprises amplifying one or more of said different sequence portions of the microbial nucleic acid and said one or more quantitative standard nucleic acids with the same primer pair.

On the other hand, it can be favorable to have different primer pairs amplify the different target sequences and the quantitative standard nucleic acid or acids. One example is the presence of very high concentrations of nucleic acids in the reaction mixture having added the biological sample, since, e.g., if the same primers confer elongation of multiple sequence portions of the target and/or the quantitative standard nucleic acid or acids. In the latter case, the concentration of said primers decreases more rapidly than if the primers only amplified one distinct sequence portion. Therefore, in a preferred embodiment, the method according to the invention comprises amplifying said different sequence portions of the microbial nucleic acid and said one or more quantitative standard nucleic acids with different primer pairs.

In order to enhance the detectable signal or signals generated by said two or more different sequence portions of the microbial nucleic acid, they can generate a signal via the same fluorescent dye. Thus, in a preferred embodiment, the method according to the invention comprises employing one fluorescent dye as a detectable label for the amplification products derived from said two or more different sequence portions of the microbial nucleic acid, and a different fluorescent dye as a detectable label for the amplification product or products derived from said one or more quantitative standard nucleic acids.

It can further be favorable to be able to determine the respective quantities of said two or more different sequence portions of the microbial nucleic acid, e.g. in order to analyze mutations or different subtypes. Therefore, in a preferred embodiment, the method according to the invention comprises employing one distinct fluorescent dye as a detectable label for each one of the amplification products derived from the microbial nucleic acid, and a different fluorescent dye as a detectable label for the amplification product or products derived from said one or more quantitative standard nucleic acids, wherein the quantifying step comprises separately quantifying each of the amplified sequence portions of the microbial target sequence.

As described above, in the TaqMan format, during the annealing step of the PCR reaction, the labeled hybridization probe binds to the target nucleic acid (i.e., the amplification product) and is degraded by the 5' to 3' exonuclease activity of the Taq or another suitable polymerase as known by the skilled artisan, such as the preferred ZO5 polymerase, during the subsequent elongation phase.

Thus, in a preferred embodiment, in the method according to the invention, amplification employs a polymerase enzyme having 5' to 3' exonuclease activity.

It can further be advantageous to employ only one quantitative standard nucleic acid for the detection and quantification of the microbial nucleic acid, as it e.g. reduces the need of resources and design of further appropriate sequences.

Therefore, preferably, in the method according to the invention, exactly one quantitative standard nucleic acid is present in the reaction mixture.

The method according to the invention can be readily applied to detect the nucleic acids derived from multiple microorganisms, thereby widening the possibilities for a use in a clinical environment with an easy single-tube detection and quantification procedure.

Thus, in a preferred embodiment, the method according to the invention comprises simultaneously detecting and quantifying said microbial nucleic acid in multiple different microorganisms.

The method according to the invention can be advantageously applied on viral nucleic acids. Thus, in a preferred embodiment of the invention, the microbial nucleic acid is a viral nucleic acid.

Among viral nucleic acids, the method according to the invention can be most advantageously be applied on HIV. Thus, in a preferred embodiment of the invention, the microbial nucleic acid is a nucleic acid of HIV.

Suitable genetic regions as detection and quantification targets within HIV are e.g. GAG, POL, and/or LTR. Most preferably, in the method according to the invention, the different sequence portions are sequences of GAG and LTR of HIV.

An example how to perform calculation of quantitative results in the TaqMan format based on an internal standard is described in the following. A titer is calculated from input data of instrument-corrected fluorescence values from an entire PCR run. A set of samples containing a target nucleic acid such as a microbial nucleic acid and a quantitative standard nucleic acid undergo PCR on a thermocycler using a temperature profile as specified. At selected temperatures and times during the PCR profile samples are illuminated by filtered light and the filtered fluorescence data are collected for each sample for the target nucleic acid and the quantitative standard nucleic acid. After a PCR run is complete, the fluorescence readings are processed to yield one set of dye concentration data for the quantitative standard nucleic acid and one set of dye concentration data for the target nucleic acid. Each set of dye concentration data are processed in the same manner. After several plausibility checks, the elbow values (CT) are calculated for the quantitative standard nucleic acid and the target nucleic acid. The elbow value is defined as the point where the fluorescence of the target nucleic acid or the quantitative standard nucleic acid crosses a predefined threshold (fluorescence concentration). Titer determination is based on the assumptions that the target nucleic acid and the quantitative standard nucleic acid are amplified with the same efficiency and that at the calculated elbow value equal amounts of amplicon copies of target nucleic acid and quantitative standard nucleic acid are amplified and detected. Therefore, the (CTQS - CTtarget) is linear to log (target conc / QS conc). The titer T can then be calculated for instance by using a polynomial calibration formula as in the following equation:

$$T' = 10^{(a(CTQS - CTtarget)2 + b(CTQS - CTtarget) + c)}$$

The polynomial constants and the concentration of the quantitative standard nucleic acid are known, therefore the only variable in the equation is the difference (CTQS - CTtarget).

Another subject of the invention is the use of a quantitative standard nucleic acid for detecting and quantifying a microbial nucleic acid according to any of the methods described above.

Further, the invention provides a kit for detecting and quantifying a microbial nucleic acid in a biological sample by any of the methods described above, said kit comprising one or more quantitative standard nucleic acids, two or more primer pairs, said primer pairs being specific for different sequence portions of said microbial nucleic acid, and one or more primer pairs specific for said one or more standard nucleic acids.

Preferably the kit additionally comprises two or more probes specific for different sequence portions of said microbial nucleic acid, and one or more probes specific for said one or more standard nucleic acids.

Such kits known in the art further comprise plastics ware which can be used during the sample preparation procedure as e.g. microtiter plates in the 96 or 384 well format or ordinary reaction tubes manufactured e.g. by Eppendorf, Hamburg, Germany and all other reagents for carrying out the method according to the invention. Therefore, the kit can additionally contain a material with an affinity to nucleic acids, preferably the material with an affinity to nucleic acids comprises a material with a silica surface. Preferably, the material with a silica surface is a glass. Most preferably, the material with an affinity to nucleic acids is a composition comprising magnetic glass particles. The kit can further or additionally comprise a lysis buffer containing e.g. chaotropic agents, detergents or alcohols or mixtures thereof allowing for the lysis of cells. These components of the kit according to the invention may be provided separately in tubes or storage containers. Depending on the nature of the components, these may be even provided in a single tube or storage container. The kit may further or additionally comprise a washing solution which is suitable for the washing step of the magnetic glass particles when a nucleic acid is bound thereto. This washing solution may contain ethanol and/ or chaotropic agents in a buffered solution or solutions with an acidic pH without ethanol and/ or chaotropic agents as described above. Often the washing solution or other solutions are provided as stock solutions which have to be diluted before use. The kit may further or additionally comprise an eluent or elution buffer, i.e. a solution or a buffer (e.g. 10 mM Tris, 1 mM EDTA, pH 8.0) or pure water to elute the nucleic acid bound to the magnetic glass particles. Further, additional reagents or buffered solutions may be present which can be used for the purification process of a nucleic acid. Preferably, the kit contains a polymerase enzyme having 5' to 3' exonuclease activity. Also preferred is that the kit contains an enzyme with reverse transcriptase activity.

In a preferred embodiment of the invention, the method according to the invention is embedded in a sequence of methods carried out within an analytical system, thereby preferably forming an automatable process.

According to the invention, such an analytical system for performing the method of the invention preferably comprises

- a sample preparation module comprising a lysis buffer for providing a biological sample
- an amplification and detection module comprising a reaction receptacle in which the method is performed.

The sample preparation module can advantageously comprise components for the sample preparation procedures supra, i.e. for example magnetic glass particles and a magnet for separating them from the solution, chaotropic salt solutions and one or more vessels that contain the crude sample and the reagents required for sample preparation.

The reaction receptacle in the amplification and detection module can for example be a microtiter plate, a centrifugation vial, a lysis tube, or any other type of vessel suitable for containing a reaction mixture according to the invention.

In a preferred embodiment of the invention, the analytical system contains a storage module containing the reagents for performing the method of the invention.

Said storage module can further contain other components useful for the method of the invention, e.g. disposables such as pipet tips or even vessels to be used as reaction receptacles within the amplification and detection module.

Furthermore, in a preferred embodiment of the invention, the analytical system contains a transfer module for transferring the biological sample from the sample preparation module to the amplification and detection module.

Even though it is possible to carry out said transfer manually, it is preferable to use an automated system wherein the transfer is performed e.g. by a robotic device such e.g. as a motor-driven mobile rack or robotic pivot arm.

Automatable process means that the steps of the process are suitable to be carried out with an apparatus or machine capable of operating with little or no external control or influence by a human being. Automated method means that the steps of the automatable method are carried out with an apparatus or machine capable of operating with little or no external control or influence by a human being. Only the preparation steps for the method may have to be done by hand, e.g. storage containers have to be filled and put into place, the choice of samples has to be performed by a human being and further steps known to the expert in the field, e.g. the operation of a controlling computer. The apparatus or machine may e.g. automatically add liquids, mix the samples or carry out incubation steps at specific temperatures. Typically, such a machine or apparatus is a robot controlled by a computer which carries out a program in which the single steps and commands are specified.

All other preferred embodiments and specific descriptions of embodiments of the uses, kits and analytical systems according to the invention are those mentioned for the method according to the invention.

FIGURE LEGENDS

Figure 1:

Frequencies of underquantitation of HIV-1 specimens in Test 2 (COBAS AmpliPrep / COBAS TaqMan HIV-1) compared to Test 1(COBAS AMPLICOR HIV-1 MONITOR) as observed in different studies performed at sites in several European countries as well as at a site in Thailand.

Figure 2:

A. Typical examples for mismatches at the 3'end of an upstream primer in an HIV-1 Real-time PCR Test leading to underquantitation of various degree as shown in column 5.

B. Typical examples for mismatches at the 3'end of a downstream primer in an HIV-1 Real-time PCR Test leading to underquantitation of various degree as shown in column 5.

Figure 3:

A number of 16 samples underquantitated in Test 2 (GAG only) if compared to Test 1 (for log10 difference in titer see column 5) was tested with the modified Test 2 which targets the GAG and the LTR regions of HIV simultaneously. The log10 titer results of Test 2 modified (GAG+LTR) were compared to Test 2 (GAG). All samples previously underquantitated in Test 2 (GAG) were quantitated significantly higher with Test 2 modified (GAG+LTR). The log10 titer results of Test 2 modified (GAG+LTR) compared to Test 1 revealed that all previously underquantitated samples were restored in titer compared to Test 1 and further identified two samples underquantitated in Test 1 (2B13 and 2B21).

Figure 4:

The limit of detection (LOD) was determined by testing several concentration levels of HIV WHO Standard below and above the expected LOD in multiple replicates. The respective LOD was determined by PROBIT analysis at 95% hitrate. Test 2 contained primers and probe for the GAG region only of HIV, Test 2a contained primers and probe for the LTR region only of HIV and Test 2 modified contained primers and probe for both the GAG and LTR regions of HIV.

## EXAMPLES

The following examples describe embodiments in which the invention can be carried out. It is clear to the person skilled in the art that these examples are not limiting and can be modified within the spirit of the invention.

### Example 1:

The test COBAS AmpliPrep / COBAS TaqMan HIV-1 (Test 2) is compared to the test COBAS AMPLICOR HIV-1 MONITOR (Test 1, both tests available from Roche Diagnostics GmbH, Mannheim, Germany) in the context of different studies performed at sites in several European countries as well as at a site in Thailand.

The tests were performed according to the manufacturer's instructions.

In brief, for Test 2, sample preparation was carried out using magnetic glass particles and chaotropic reagents, while in the amplification and detection step, the following concentrations were employed:

Primers directed towards the GAG region: 0.003%

Probes specific for the amplified products of GAG and the quantitative standard nucleic acid: 0.003%

ZO5 DNA Polymerase: 0.05%

dUTP, dATP, dTTP, dCTP, dTTP: 0.04%

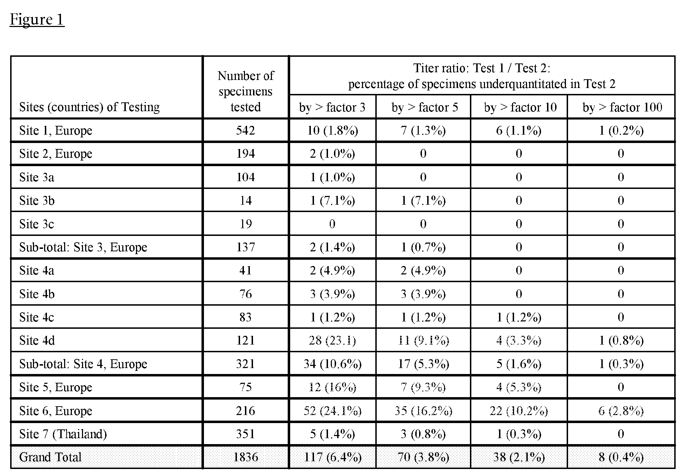

The concentrations were determined by PROBIT analysis at 95 % hitrate, the results are shown in Figure 1.

### Example 2:

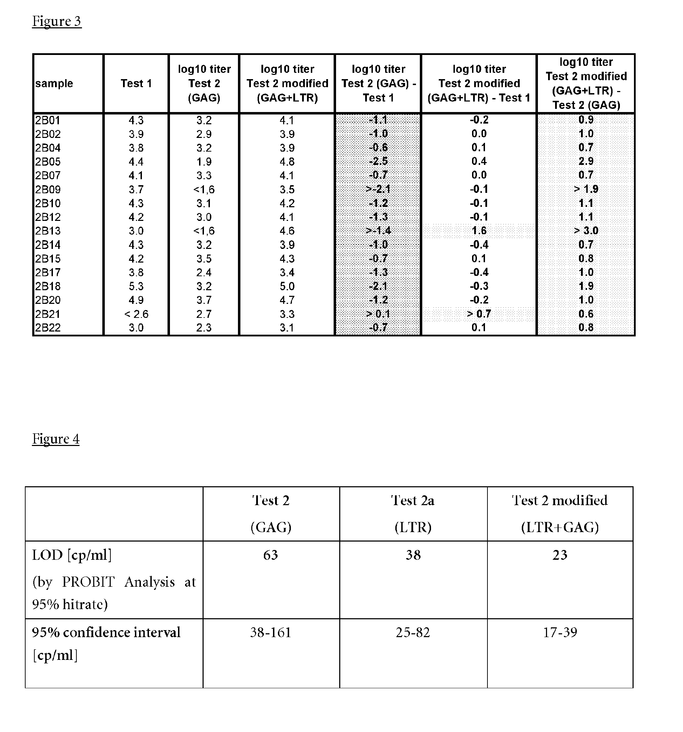

A number of 16 samples underquantitated in Test 2 (COBAS TaqMan HIV-1, targets GAG only) if compared to Test 1 (for log10 difference in titer see column 5) was tested with the modified Test 2 which targets the GAG and the LTR regions of HIV simultaneously. The results are displayed in Figure 3. Test conditions were the same for both tests, with the exception that additional primers and a probe for LTR were introduced at about a third of the concentration of the oligonucleotides for amplification and detection of GAG.

Example 3:

In essentially the same setting as for Example 2, the LOD was determined for Test 2 and modified Test 2. Additionally, a third experiment was carried out with the oligonucleotides for amplification and detection of LTR but not of GAG (Test 2a). The oligonucleotide concentration in Test 2a was equivalent to the concentration of oligonucleotides for amplification and detection of LTR in modified Test 2.

## Claims

1. A method for detecting and quantifying a microbial nucleic acid in a biological sample wherein said microbial nucleic acid is a nucleic acid of HIV, said method comprising:

   a) providing a reaction mixture comprising one or more quantitative standard nucleic acids, two or more primer pairs, said primer pairs being specific for different sequence portions of said microbial nucleic acid, and one or more primer pairs specific for said one or more standard nucleic acids
   b) adding said biological sample to said reaction mixture
   c) performing one or more cycling steps, wherein a cycling step comprises an amplifying step, said amplifying step comprising producing two or more different amplification products derived from said microbial nucleic acid if present in said sample and producing one or more amplification products derived from said one or more quantitative standard nucleic acids
   d) detecting and measuring detectable signals generated by said amplification products and proportional to their concentration in said reaction mixture, wherein the presence or absence of the detectable signal or signals generated by said microbial nucleic acid is indicative of the presence or absence of the microbial nucleic acid in said biological sample
e) determining the quantity of said microbial nucleic acid in said biological sample by comparison of the signals generated by said microbial nucleic acid and said one or more quantitative standard nucleic acids, wherein at least one of said two or more different sequence portions is a sequence of GAG, POL or LTR of HIV.

**2.** The method of claim 1, additionally comprising

in step a) providing two or more probes specific for the different sequence portions amplified by said two or more primer pairs specific for different sequence portions of said microbial nucleic acid, and one or more probes specific for the sequence portion or portions amplified by said one or more primer pairs specific for said one or more quantitative standard nucleic acids.

**3.** The method of claim 2, additionally comprising

in step c) a hybridizing step comprised by said cycling step, wherein said hybridizing step comprises hybridizing the different sequence portions amplified by said two or more primer pairs specific for different sequence portions of said microbial nucleic acid, if the microbial nucleic acid is present in said sample, with said two or more probes, and hybridizing said sequence portion or portions amplified by said one or more primer pairs specific for said one or more quantitative standard nucleic acids with one or more probes, wherein the probes are labeled with a donor fluorescent moiety and a corresponding acceptor fluorescent moiety
in step d) detecting the presence or absence of fluorescence resonance energy transfer (FRET) between said donor fluorescent moiety and said acceptor fluorescent moiety of said probe, wherein the presence or absence of fluorescence generated by said microbial nucleic acid is indicative of the presence or absence of the microbial nucleic acid in said biological sample
in step e) determining the quantity of said microbial nucleic acid in said sample by comparison of the fluorescent signals generated by said microbial nucleic acid and said quantitative standard nucleic acid.

**4.** The method of any of the preceding claims, comprising amplifying one or more of said different sequence portions of the microbial nucleic acid and said one or more quantitative standard nucleic acids with the same primer pair.

**5.** The method of any of the preceding claims, comprising amplifying said different sequence portions of the microbial nucleic acid and said one or more quantitative standard nucleic acids with different primer pairs.

**6.** The method of any of the preceding claims, comprising employing one fluorescent dye as a detectable label for the amplification products derived from said two or more different sequence portions of the microbial nucleic acid, and a different fluorescent dye as a detectable label for the amplification product or products derived from said one or more quantitative standard nucleic acids.

**7.** The method of any of the claims 1-5, comprising employing one distinct fluorescent dye as a detectable label for each one of the amplification products derived from the microbial nucleic acid, and a different fluorescent dye as a detectable label for the amplification product or products derived from said one or more quantitative standard nucleic acids, wherein the quantifying step comprises separately quantifying each of the amplified sequence portions of the microbial target sequence.

**8.** The method of any of the preceding claims, wherein exactly one quantitative standard nucleic acid is present in the reaction mixture.

**9.** The method of any of the preceding claims, wherein the nucleic acid sequences of said one or more primers are at least 12 contiguous nucleotides selected from the group of SEQ ID NO 1-16 and 21-27 or the corresponding complementary nucleic acid sequences thereof.

**10.** The method of any of the claims 2 or 3, wherein the nucleic acid sequences of said two or more probes are at least 12 contiguous nucleotides selected from the group of SEQ ID NO 17-20, 28, 29 or the corresponding complementary nucleic acid sequences thereof.

**11.** Use of a quantitative standard nucleic acid for detecting and quantifying a microbial nucleic acid, said microbial nucleic acid being a nucleic acid of HIV, according to the method of any of the preceding claims.

**12.** A kit for detecting and quantifying a microbial nucleic acid, said microbial nucleic acid being a nucleic acid of HIV, in a biological sample by the method of any of the preceding claims, said kit comprising one or more quantitative standard nucleic acids, two or more primer pairs, said primer pairs being specific for different sequence portions of said microbial nucleic acid, and one or more primer pairs specific for said one or more standard nucleic acids, wherein at least one of said two or more different sequence portions is a sequence of GAG, POL or LTR of HIV.

Figure 1

| Sites (countries) of Testing | Number of specimens tested | Titer ratio: Test 1 / Test 2: percentage of specimens underquantitated in Test 2 | | | |
|---|---|---|---|---|---|
| | | by > factor 3 | by > factor 5 | by > factor 10 | by > factor 100 |
| Site 1, Europe | 542 | 10 (1.8%) | 7 (1.3%) | 6 (1.1%) | 1 (0.2%) |
| Site 2, Europe | 194 | 2 (1.0%) | 0 | 0 | 0 |
| Site 3a | 104 | 1 (1.0%) | 0 | 0 | 0 |
| Site 3b | 14 | 1 (7.1%) | 1 (7.1%) | 0 | 0 |
| Site 3c | 19 | 0 | 0 | 0 | 0 |
| Sub-total: Site 3, Europe | 137 | 2 (1.4%) | 1 (0.7%) | 0 | 0 |
| Site 4a | 41 | 2 (4.9%) | 2 (4.9%) | 0 | 0 |
| Site 4b | 76 | 3 (3.9%) | 3 (3.9%) | 0 | 0 |
| Site 4c | 83 | 1 (1.2%) | 1 (1.2%) | 1 (1.2%) | 0 |
| Site 4d | 121 | 28 (23.1) | 11 (9.1%) | 4 (3.3%) | 1 (0.8%) |
| Sub-total: Site 4, Europe | 321 | 34 (10.6%) | 17 (5.3%) | 5 (1.6%) | 1 (0.3%) |
| Site 5, Europe | 75 | 12 (16%) | 7 (9.3%) | 4 (5.3%) | 0 |
| Site 6, Europe | 216 | 52 (24.1%) | 35 (16.2%) | 22 (10.2%) | 6 (2.8%) |
| Site 7 (Thailand) | 351 | 5 (1.4%) | 3 (0.8%) | 1 (0.3%) | 0 |
| Grand Total | 1836 | 117 (6.4%) | 70 (3.8%) | 38 (2.1%) | 8 (0.4%) |

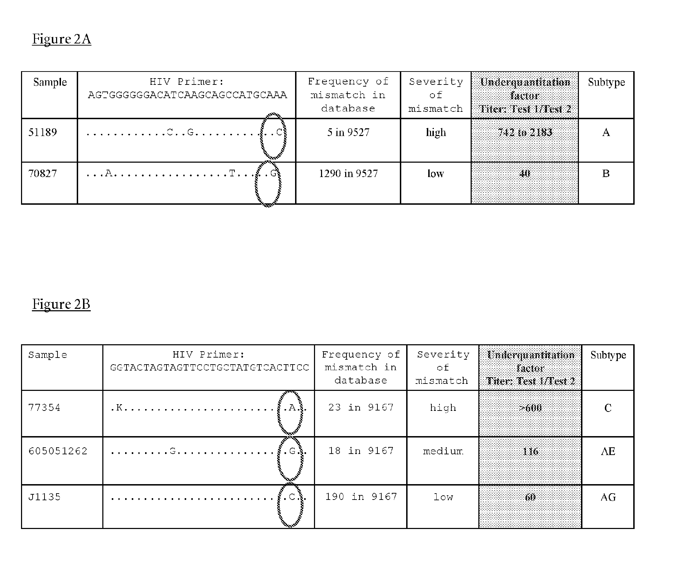

Figure 2A

| Sample | HIV Primer: AGTGGGGGGACATCAAGCAGCCATGCAAA | Frequency of mismatch in database | Severity of mismatch | Underquantitation factor Titer: Test 1/Test 2 | Subtype |
|---|---|---|---|---|---|
| 51189 | ............C..G............C | 5 in 9527 | high | 742 to 2183 | A |
| 70827 | ...A.................T.....G | 1290 in 9527 | low | 40 | B |

Figure 2B

| Sample | HIV Primer: GGTACTAGTAGTTCCTGCTATGTCACTTCC | Frequency of mismatch in database | Severity of mismatch | Underquantitation factor Titer: Test 1/Test 2 | Subtype |
|---|---|---|---|---|---|
| 77354 | .K.........................A.. | 23 in 9167 | high | >600 | C |
| 605051262 | .........G.................G.. | 18 in 9167 | medium | 116 | AE |
| J1135 | ...........................C.. | 190 in 9167 | low | 60 | AG |

Figure 3

| sample | Test 1 | log10 titer Test 2 (GAG) | log10 titer Test 2 modified (GAG+LTR) | log10 titer Test 2 (GAG) - Test 1 | log10 titer Test 2 modified (GAG+LTR) - Test 1 | log10 titer Test 2 modified (GAG+LTR) - Test 2 (GAG) |
|---|---|---|---|---|---|---|
| 2B01 | 4.3 | 3.2 | 4.1 | -1.1 | -0.2 | 0.9 |
| 2B02 | 3.9 | 2.9 | 3.9 | -1.0 | 0.0 | 1.0 |
| 2B04 | 3.8 | 3.2 | 3.9 | -0.6 | 0.1 | 0.7 |
| 2B05 | 4.4 | 1.9 | 4.8 | -2.5 | 0.4 | 2.9 |
| 2B07 | 4.1 | 3.3 | 4.1 | -0.7 | 0.0 | 0.7 |
| 2B09 | 3.7 | <1,6 | 3.5 | >-2.1 | -0.1 | > 1.9 |
| 2B10 | 4.3 | 3.1 | 4.2 | -1.2 | -0.1 | 1.1 |
| 2B12 | 4.2 | 3.0 | 4.1 | -1.3 | -0.1 | 1.1 |
| 2B13 | 3.0 | <1,6 | 4.6 | >-1.4 | 1.6 | > 3.0 |
| 2B14 | 4.3 | 3.2 | 3.9 | -1.0 | -0.4 | 0.7 |
| 2B15 | 4.2 | 3.5 | 4.3 | -0.7 | 0.1 | 0.8 |
| 2B17 | 3.8 | 2.4 | 3.4 | -1.3 | -0.4 | 1.0 |
| 2B18 | 5.3 | 3.2 | 5.0 | -2.1 | -0.3 | 1.9 |
| 2B20 | 4.9 | 3.7 | 4.7 | -1.2 | -0.2 | 1.0 |
| 2B21 | < 2.6 | 2.7 | 3.3 | > 0.1 | > 0.7 | 0.6 |
| 2B22 | 3.0 | 2.3 | 3.1 | -0.7 | 0.1 | 0.8 |

Figure 4

| | Test 2 (GAG) | Test 2a (LTR) | Test 2 modified (LTR+GAG) |
|---|---|---|---|
| LOD [cp/ml] (by PROBIT Analysis at 95% hitrate) | 63 | 38 | 23 |
| 95% confidence interval [cp/ml] | 38-161 | 25-82 | 17-39 |

Europäisches Patentamt
European Patent Office
Office européen des brevets

# EUROPEAN SEARCH REPORT

Application Number
EP 11 18 1688

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HUANG J ET AL: "A novel real-time multiplex reverse transcriptase-polymerase chain reaction for the detection of HIV-1 RNA by using dual-specific armored RNA as internal control", INTERVIROLOGY 200804 CH, vol. 51, no. 1, April 2008 (2008-04), pages 42-49, XP009110430, ISSN: 0300-5526 * abstract * * page 43, column 1, paragraph 2 * * page 43, column 2, paragraph 2 - page 45, column 1, paragraph 3 * * page 46, column 1, paragraph 1 * * page 46, column 2, paragraph 1 - page 47, column 1, paragraph 1; figures 1-3; tables 1-4 * ----- | 1-11 | INV. C12Q1/68 |
| X | WO 95/14109 A (US ARMY [US]) 26 May 1995 (1995-05-26) * page 9, lines 12-24 * ----- | 12 | |
| | | | TECHNICAL FIELDS SEARCHED (IPC) |
| A | MEREL ET AL: "Perspectives on Molecular Diagnostics Automation", JOURNAL OF THE ASSOCIATION FOR LABORATORY AUTOMATION, ELSEVIER, vol. 10, no. 5, 1 October 2005 (2005-10-01), pages 342-350, XP005445580, ISSN: 1535-5535 * abstract; tables I,II * * page 343, column 2, paragraph 2 - page 346, column 1, paragraph 4 * * page 348, column 1, paragraph 3 - column 2, paragraph 3 * * page 350 * ----- -/-- | 1-12 | C12Q |

The present search report has been drawn up for all claims

1

| Place of search | Date of completion of the search | Examiner |
|---|---|---|
| Munich | 23 November 2011 | Tilkorn, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 11 18 1688

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | DROSTEN CHRISTIAN ET AL: "Ultrasensitive monitoring of HIV-1 viral load by a low-cost real-time reverse transcription-PCR assay with internal control for the 5' long terminal repeat domain.", CLINICAL CHEMISTRY JUL 2006, vol. 52, no. 7, July 2006 (2006-07), pages 1258-1266, XP002509656, ISSN: 0009-9147 * the whole document * ----- | 1-12 | |
| A | MADHAVAN ET AL: "Development and application of a novel multiplex polymerase chain reaction for semi-quantitation of Human Cytomegalovirus in clinical specimens", JOURNAL OF VIROLOGICAL METHODS, ELSEVIER BV, NL, vol. 141, no. 2, 27 March 2007 (2007-03-27), pages 166-172, XP022002021, ISSN: 0166-0934 * the whole document * ----- | 1-12 | TECHNICAL FIELDS SEARCHED (IPC) |
| A | EP 1 602 736 A (BIO RAD PASTEUR [FR]) 7 December 2005 (2005-12-07) * example 5 * ----- | 1-12 | |
| A | CANDOTTI DANIEL ET AL: "Multiplex real-time quantitative RT-PCR assay for hepatitis B virus, hepatitis C virus, and human immunodeficiency virus type 1.", JOURNAL OF VIROLOGICAL METHODS 1 JUN 2004, vol. 118, no. 1, 1 June 2004 (2004-06-01), pages 39-47, XP002509657, ISSN: 0166-0934 * the whole document * ----- | 1-12 | |

1

The present search report has been drawn up for all claims

| Place of search | Date of completion of the search | Examiner |
|---|---|---|
| Munich | 23 November 2011 | Tilkorn, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 18 1688

This annex lists the patent family members relating to the patent documents cited in the above-mentioned European search report.
The members are as contained in the European Patent Office EDP file on
The European Patent Office is in no way liable for these particulars which are merely given for the purpose of information.

23-11-2011

| Patent document cited in search report | Publication date | Patent family member(s) | Publication date |
|---|---|---|---|
| WO 9514109 A | 26-05-1995 | AU 1259295 A | 06-06-1995 |
| | | WO 9514109 A1 | 26-05-1995 |
| EP 1602736 A | 07-12-2005 | AT 452996 T | 15-01-2010 |
| | | AU 2005250146 A1 | 15-12-2005 |
| | | CA 2567007 A1 | 15-12-2005 |
| | | EP 1602736 A1 | 07-12-2005 |
| | | EP 1751317 A1 | 14-02-2007 |
| | | ES 2336807 T3 | 16-04-2010 |
| | | JP 2008501328 A | 24-01-2008 |
| | | US 2008318205 A1 | 25-12-2008 |
| | | WO 2005118883 A1 | 15-12-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20040229211 A
- US 4458066 A
- WO 0212263 A
- US 4683202 A
- US 4683195 A
- US 4800159 A
- US 4965188 A
- WO 9001069 A
- EP 0439182 A2
- WO 9208808 A
- US 5130238 A
- WO 9804730 A
- US 5386024 A
- EP 1201753 A
- DE 3734442 A
- GB 9100212 W
- WO 0137291 A
- US 5035996 A
- US 5683896 A
- US 5945313 A
- WO 9746707 A
- WO 9746714 A
- WO 9746712 A


### Non-patent literature cited in the description


- **SIDDAPPA et al.** *J. Clin. Microbiol.,* 2004, vol. 42, 2742-2751
- **HERRMANN et al.** *J. Clin. Microbiol.,* 2004, vol. 42, 1909-1914
- **SAMBROOK J. et al.** Molecular Cloning: A Laboratory Manual. Cold Spring Harbor Laboratory Press, 1984
- Nucleic Acid Hybridization. Methods in Enzymology. Academic Press, Inc, 1984
- **NARANG S. A. et al.** *Methods in Enzymology,* 1979, vol. 68, 90-98
- **BROWN E. L. et al.** *Methods in Enzymology,* 1979, vol. 68, 109-151
- **BEAUCAGE et al.** *Tetrahedron Letters,* 1981, vol. 22, 1859
- **GAREGG et al.** *Chem. Scr.,* 1985, vol. 25, 280-282
- **UHLMANN ; PEYMAN.** *Chemical Reviews,* 1990, vol. 90, 543
- **VERMA S. ; ECKSTEIN F.** *Annu. Rev. Biochem.,* 1998, vol. 67, 99-134
- **WU D. Y. ; WALLACE R. B.** *Genomics,* 1989, vol. 4, 560-69
- **BARANY F.** *Proc. Natl. Acad. Sci. USA,* 1991, vol. 88, 189-193
- **BARANY F.** *PCR Methods and Applic.,* 1991, vol. 1, 5-16
- **KWOH D. Y. et al.** *Proc. Natl. Acad. Sci. USA,* 1989, vol. 86, 1173-1177
- **GUATELLI J.C. et al.** *Proc. Natl. Acad. Sci. USA,* 1990, vol. 87, 1874-1878
- **WHELEN A. C. ; PERSING D. H.** *Annu. Rev. Microbiol.,* 1996, vol. 50, 349-373
- **ABRAMSON R. D. ; MYERS T. W.** *Curr Opin Biotechnol,* 1993, vol. 4, 41-47
- **SAMBROOK J. et al.** Molecular Cloning: A Laboratory Manual. Cold Spring Harbor Laboratory Press, 1989
- **AUSUBEL F. et al.** Current Protocols in Molecular Biology. J. Wiley and Sons, 1987
- **WALSH.** Enzymatic Reaction Mechanisms. W. H. Freeman and Company, 1979
- **VOGELSTEIN B. et al.** *Proc. Natl. Acad. USA,* 1979, vol. 76, 615-9
- **MARKO M. A. et al.** *Anal. Biochem.,* 1982, vol. 121, 382-387
- **JAKOBI R. et al.** *Anal. Biochem.,* 1988, vol. 175, 196-201
- **ALDERTON R. P. et al.** *S., Anal. Biochem.,* 1992, vol. 201, 166-169
- **R. BOOM et al.** *J Clin Microbiol.,* 1990, vol. 28, 495-503